# EUROPEAN PATENT APPLICATION

(11) **EP 3 406 670 A1**
(43) Date of publication of application: **28.11.2018**
(21) Application number: 17741727.6
(22) Date of filing: 09.01.2017
(51) Int. Cl.: C09B 47/22, C07D 487/22, C07F 1/08

(54) **SUBSTANCE WHICH ABSORBS ELECTROMAGNETIC RADIATION IN THE NEAR-INFRARED REGION OF THE SPECTRUM, AND METHOD OF PRODUCING SAME**

(30) Priority: 22.01.2016 RU 2016102078
(71) Applicant: Matiushin, Gennadii Alekseevich, Moscow 117463 (RU); Tribelskiy, Mikhail Isaakovich, Moskovskaya obl. 141802 (RU)
(72) Inventor: BELYAKOV, Nikolay Grigorivich, Moscow 127549 (RU); LUKYANEC, Evgeny Antonovich, Moscow 127238 (RU); MATIUSHIN, Gennadii Alekseevich, Moscow 127238 (RU); TRIBELSKIY, Mikhail Isaakovich, Moscow 127238 (RU)
(74) Representative: Benatov, Samuil Gabriel
(86) International application number: PCT/RU2017/000004
(87) International publication number: WO 2017/126993

(57) **Abstract**

A substance with absorption in the near infrared region is proposed. The substance is a mixture of compounds with various degree of substitution of chlorine for amino group in chlorinated copper phthalocyanine obtained by means of amination of a green phthalocyanine coloring agent at least by one of the following compounds taken in proportion from 5 to 120 parts per one part of the green phthalocyanine coloring agent by weight: diethyl amine, dibutylamine, piperidine, morpholine, piperazine. We also propose a method for production of the substance, which consists in amination of the green phthalocyanine coloring agent (chlorinated copper phthalocyanine), at least by one of compounds from the group, taken in proportion from 5 to 120 parts per one part of the green phthalocyanine coloring agent by weight: diethyl amine, dibutylamine, piperidine, morpholine, piperazine at a temperature from 100 to 300° C and pressure from 5 to 30 atm, being carried out until a substance with absorption maximum at 760-810 nm is obtained.

## Description

The invention relates to the sphere of chemistry, namely, production of substances, coloring agents and compounds which can be used as a substance absorbing electromagnetic radiation in the near infrared region - including subsequent production of polymer and liquid materials for various purposes which change the spectral content of transmitted and reflected radiation, e. g., in cut-off light filters.

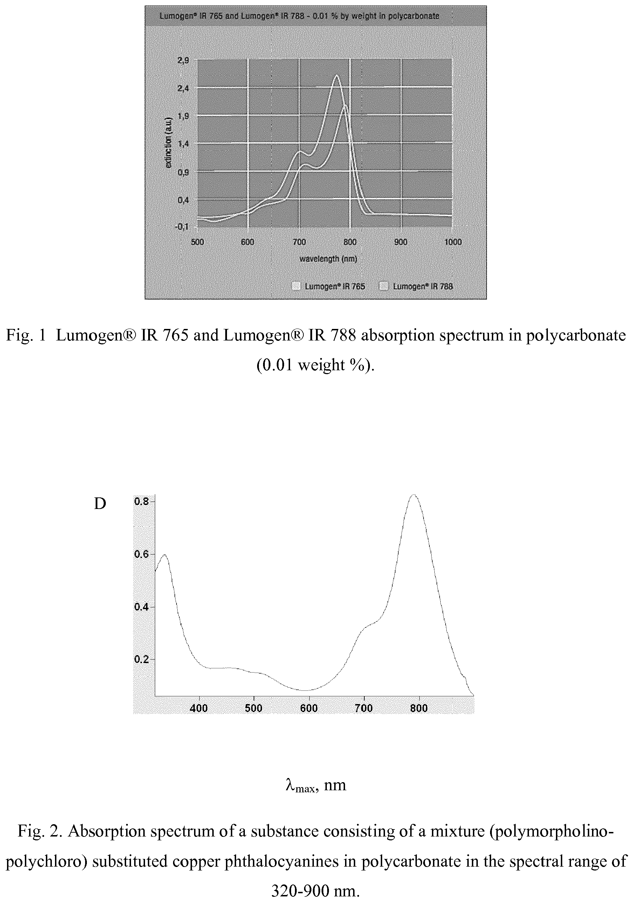

A set of coloring agents of the Lumogen group absorbing in the near infrared region is known, manufactured by BASF (Langhals H. Control of the Interactions in Multichromophores: NovelConcepts. Perylene Bis - imides as Components for Larger Functional Units. Helvetica chimica acta. - 2005. - V. 88. No. 6. - P. 1309-1343). Thus, Lumogen® IR 788 and Lumogen® IR 765 are derivatives of quaterrylene bis(dicarboximide) and they absorb in the region of 750-800 nm (fig. 1).

A disadvantage of these compounds usually used for laser welding of polymers is due to their luminescent properties which significantly limit their area of application as an additive for cut-off light filters.

A large number of polymethine (cyanine) coloring agents, whose absorption spectra overlap a significant part of the near-infrared region, is known; however, all of them are not sufficiently stable and not easily accessible (A.A. Ischenko. Architecture and spectral-luminescent properties of polymethine dyes. Naukova dumka, Kiev, 1994).

Derivatives of tetra-1-phenyl-2,3-naphthalocyanine, adsorbing in the region of 780-850 nm (Galpern M.G., Goncharov G.I., Kovshev E.I., Lukyanets E.A., Seliverstov V.A., Tsvetkov V.A. Metallic complexes of tetra-1-phenyl-2,3-naphthalocyanine as components of IR filters in liquid-crystal matrixes. Author certificate No. 921239, 1981), are known. However, they do not have a good coefficient of solubility in polymer matrixes and are not easily accessible.

Derivatives of phthalocyanine, such as dialkylamides of phthalocyaninetetrasulfoacids, are more easily accessible and they are well introduced into polymers; however, polymer matrixes produced with application thereof adsorb in the red area (670-720 nm) only (Solovyeva L.I. Mikhalenko S.A., Chernykh E.V., Lukyanets E.A. Russian Journal of General Chemistry, 52, 90, 1982).

An invention patent (Fedotova A.I., Maizlish V.E., Shaposhnikov G.P. RF patent No. 2354657, 2009), where substituted copper metal phthalocyanines-tetra-4-(morpholine-4-yl)-tetra-5-(phenoxy) phthalocyanine, which has intensive absorption bands with maximum at 697 nm in chloroform and at 637, 688 nm (1:0,71) in dimethyl formamide, is claimed. However, the complex produced according to this method does not have the required absorption in the near infrared region.

The desirable technical result of the proposed solution is obtaining a non-luminescent substance which has an intensive absorption band with maximum at 760 - 810 nm, negligible absorption in the visible section of the spectrum, decomposition temperature of more than 300 °C, and which could be introduced into polymers in larger quantities - a substance suitable for application in products during the processing of polymer raw materials by means of the casting method under pressure and extrusion molding.

Thereat, it would be desirable that the substance is available for production of polymer products colored therewith on a commercial scale.

It turned out that this property package is characteristic for the new substance obtained by us as a result of one-stage amination chemical reaction, which is an undivided mixture consisting of derivatives of copper phthalocyanine, whose molecule contains also tertiary amino groups and chlorine atoms The source raw materials were commercially produced green phthalocyanine coloring agent Green7 (chlorinated phthalocyanine), diethylamine, dibutylamine (di-n-butylamine), or piperidine (pentamethyleneamine), or morpholine (tetrahydro-1,4-oxazine), or piperazine (diethylene diamine), which under usual conditions do not interact with chlorinated copper phthalocyanine and substitution of chlorine for amino groups does not take place. Nevertheless, having selected the required temperature and pressure, we were successful in carrying out amination chemical reaction where substitution of chlorine atoms for a corresponding amino group takes place. Any of the outlined combinations ensures achievement of the desired technical result and allows to obtain substances consisting of a mixture of compounds with various degree of chlorine substitution for an amino group in chlorinated copper phthalocyanine.

The result is that in all cases we obtained dark color powdery substances which have a decomposition temperature of more than 350 °C, dissolve in chloroform, dichloromethane, benzene, toluene, acids and do not dissolve in water, hexane, alcohols, and poorly dissolve in acetone. They are easily introduced into polycarbonate and polymethyl methacrylate in a concentration of more than 2 mass percent. They are suitable for commercial production of superconcentrate on the basis of polycarbonate and other polymer materials. They have absorption maximum in the region of 770-800 nm and have high resistance to light. The substance, which is a mixture (polymorpholino-polychloro) of substituted copper phthalocyanines introduced into polycarbonate film, is stable under sun light for no less than seven years.

The typical absorption spectrum of the obtained substance in polycarbonate is presented in Fig. 2.

The main advantage of these substances, as compared to the known spectral analogs, is their high absorption intensity in the near IR region, heat stability up to a temperature of 350 °C, high resistance to light and solvability in the polymer matrix, as well as absence of luminescence. The advantages of the substances include their relatively low price and availability as they are obtained by means of amination of well known and commercially produced green phthalocyanine coloring agent Green7 with diethyl amine, dibutylamine, piperidine, morpholine or piperazine, which are produced by the chemical industry in sufficient amounts.

### Example 1 of substance production

An autoclave was loaded with 10 g of green phthalocyanine coloring agent and 450 g of diethylamine (load factor of 0.75) via the charging adapter, then the charging adapter was closed with a plug. The autoclave was purged with nitrogen and its content was heated to a temperature of 250° C. In the course of heating, the pressure in the autoclave was gradually increased and the maximum pressure was 23 atm at 250° C. The mixer drive was switched on and the process of amination was carried out for 4 hours. Upon expiration of the exposure, the locking device on the lower adapter was crack opened and a sample for spectrum analysis was taken, then the locking device was closed. Having made sure that, upon appearance of the absorption band in the region of 760-810 nm, amination was successful, we switched off the mixer drive, cooled the autoclave to room temperature and unloaded the reaction mass via the lower adapter while the locking device was completely open. The reaction mass was boiled out in a rotary evaporator connected to the vacuum network while being heated in a water-bath. The diethylamine was distilled at residual pressure of 200-250 mm of mercury and water-bath temperature of 90-95° C until its complete disappearance.

The residual matter from the evaporative retort was transferred to a vacuum filter (a porcelain Buchner funnel with a Bunsen flask) equipped with a two-layer filter membrane in the form of filter paper and coarse calico. The liquid phase was filtered in the network vacuum (residual pressure 0.2-0.3 atm), the precipitation was washed out with water until complete absence of chlorine ions in the flushing water. The washed precipitate in a steel glazed vessel was placed in a dryer and dried at 90° C until reaching constant weight. The yield of 21.3%. The longwave absorption maximum in benzene is 794 nm.

### Example 2 of substance production

The autoclave was loaded with 11.76 g of green phthalocyanine coloring agent and 500 g of morpholine (the load factor of 0.8) via the charging adapter, then the charging adapter was closed with a plug. The autoclave was purged with nitrogen and its content was heated to a temperature of 250° C. In the course of heating, the pressure in the autoclave was gradually increased and the maximum pressure was 23 atm at 250° C. The mixer drive was switched on and the process of amination was carried out for 4 hours. Upon expiration of the exposure, the locking device on the lower adapter was crack opened and a sample for spectrum analysis was taken, then the locking device was closed. Having made sure that, upon appearance of an absorption band in the region of 760-810 nm, the amination was successful, we switched the mixer drive off, cooled the autoclave to room temperature and unloaded the reaction mass via the lower adapter while the locking device was completely open. The reaction mass was boiled out in a rotary evaporator connected to the vacuum network while being heated in a water-bath. The dibutylamine was distilled at residual pressure of 200-250 mm of mercury and water-bath temperature of 90-95° C until its complete disappearance.

The residual matter from the evaporative retort was transferred to a vacuum filter (a porcelain Buchner funnel with a Bunsen flask) equipped with a two-layer filter membrane in the form of filter paper and coarse calico. The liquid phase was filtered in the network vacuum (residual pressure 0.2-0.3 atm), the precipitation was washed out with water until complete absence of chlorine ions in the flushing water.

The washed precipitate in a steel glazed vessel was placed in a dryer and dried at 90° C until reaching constant weight. The yield of 43.7%. The longwave absorption maximum in benzene is 794 nm.

### Example 3 of substance production

The autoclave was loaded with 9.41 g of green phthalocyanine coloring agent and 400 g of piperidine (load factor of 0.7) via the charging adapter, then the charging adapter was closed with a plug. The autoclave was purged with nitrogen and its content was heated to a temperature of 200° C. In the course of heating, the pressure in the autoclave was gradually increased and the maximum pressure was 25 atm at 200° C. The mixer drive was switched on and the process of amination was carried out for 6 hours. Upon expiration of the exposure, the locking device on the lower adapter was crack opened and a sample for spectrum analysis was taken, then the locking device was closed.

Having made sure that, upon appearance of an absorption band in the region of 760-810 nm, the amination was successful, we switched the mixer drive off, cooled the autoclave to room temperature and unloaded the reaction mass via the lower adapter while the locking device was completely open. The reaction mass was boiled out in a rotary evaporator connected to the vacuum network while being heated in a water-bath. The piperidine was distilled at residual pressure of 200-250 mm of mercury and water-bath temperature of 80-85° C until its complete disappearance.

The sirupy residual matter then was transferred to a vacuum filter (a porcelain Buchner funnel with a Bunsen flask) equipped with a two-layer filter membrane in the form of filter paper and coarse calico. The liquid phase was filtered in the network vacuum (residual pressure 0.2-0.3 atm), the precipitation was washed out with water until complete absence of chlorine ions in the flushing water.

The washed precipitate in a steel glazed vessel was placed in a dryer and dried at 90° C until reaching constant weight. The yield of 47.3%. The longwave absorption maximum in benzene is 801 nm.

### Example 4 of substance production

The autoclave was loaded with 11.76 g of green phthalocyanine coloring agent and 500 g of morpholine (load factor of 0.8) via the charging adapter, then the charging adapter was closed with a plug. The autoclave was purged with nitrogen and its content was heated to a temperature of 250° C. In the course of heating, the pressure in the autoclave was gradually increased and the maximum pressure was 27 atm at 250° C. The mixer drive was switched on and a process of amination was carried out for 5 hours. Upon expiration of the exposure, the locking device on the lower adapter was crack opened and a sample for spectrum analysis was taken, then the locking device was closed.

Having made sure that, upon appearance of an absorption band in the region of 760-810 nm, the amination was successful, we switched the mixer drive off, cooled the autoclave to room temperature and unloaded the reaction mass via the lower adapter while the locking device was completely open. The reaction mass was boiled out in a rotary evaporator connected to the vacuum network while being heated in a water-bath. The morpholine was distilled at residual pressure of 200-250 mm of mercury and water-bath temperature of 90-95° C until its complete disappearance.

The sirupy residual matter from the evaporative retort was transferred to a vacuum filter (a porcelain Buchner funnel with a Bunsen flask) equipped with a two-layer filter membrane in the form of filter paper and coarse calico. The liquid phase was filtered in the network vacuum (residual pressure 0.2-0.3 atm), the precipitation was washed out with water until complete absence of chlorine ions in the flushing water.

The washed precipitate in a steel glazed vessel was placed in a dryer and dried at 90° C until reaching constant weight. The yield of 65.3%. The longwave absorption maximum in benzene is 797 nm.

### Example 5 of substance production

The autoclave was loaded with 12.0 g of green phthalocyanine coloring agent and 500 g of piperazine (load factor of 0.8) via the charging adapter, then the charging adapter was closed with a plug. The autoclave was purged with nitrogen and its content was heated to a temperature of 250° C. In the course of heating, the pressure in the autoclave was gradually increased and the maximum pressure was 25 atm at 250° C. The mixer drive was switched on and a process of amination was carried out for 6 hours. Upon expiration of the exposure, the locking device on the lower adapter was crack opened and a sample for spectrum analysis was taken, then the locking device was closed.

Having made sure that, upon appearance of the absorption band in the region of 760-810 nm, the amination was successful, we switched the mixer drive off, cooled the autoclave to room temperature and unloaded the reaction mass via the lower adapter while the locking device was completely open. The reaction mass was placed in a glass flat-ended conic two-liter wide-mouthed flask and water was poured therein. After thorough agitation, the water together with the nonsolute was poured on two layers of filter paper covering the flat bottom of the Buchner funnel. Then the mass left on the paper was transferred to the vacuum filter (a porcelain Buchner funnel with a Bunsen flask connected to technical vacuum at pressure of 0.2-0.3 atm) equipped with a two-layer filter membrane in the form of filter paper and coarse calico. The precipitation was washed out with water until the complete absence of chlorine ions in the flushing water.

The washed precipitate in a steel glazed vessel was placed in a dryer and dried at 90° C until reaching constant weight. The yield of 31.1%. The longwave absorption maximum in benzene is 802 nm.

### Example of substance application in the process of polycarbonate casting

Superconcentrate in the form of polycarbonate granules with the substance introduced in concentration of 2 mass percent is added into the casting machine hopper with Makrolon polycarbonate for the purpose of producing of 100x100x2 mm polymer plates. The cylinder temperature is 290 - 300° C, the mould temperature is 95° C, the injection rate is 40 cm³ per second, the molding pressure is 900 bar, the molding time is 14 seconds. Uniformly colored plates with optical quality were obtained.

### Example of substance application in the process of polymer film production

A double-screw extruder with unidirectional rotation of the screw was fed with a mixture of pure polycarbonate and superconcentrate at the a of 10 kg/h, the screw speed was 200 rpm, the extruder cylinder temperature was 300° C.

A suitable for use, uniformly colored sleeve with film thickness of 100 µm was obtained.

## Claims

1. A substance with absorption in the near infrared region, which is a mixture of compounds with various degree of substitution of chlorine for amino group in chlorinated copper phthalocyanine, obtained by means of amination of green phthalocyanine coloring agent at least by one of the following compounds taken in A proportion from 5 to 120 parts per one part of the green phthalocyanine coloring agent by weight: diethylamine, dibutylamine, piperidine, morpholine, piperazine.

2. A method for the production of a substance with absorption in the near infrared region, which consists in amination of a green phthalocyanine coloring agent (chlorinated copper phthalocyanine), at least by one of compounds from the group, taken in a proportion from 5 to 120 parts per one part of the green phthalocyanine coloring agent by weight: diethyl amine, dibutylamine, piperidine, morpholine, piperazine at a temperature from 100 to 300° C and pressure from 5 to 30 atm, being carried out until a substance with absorption maximum at 760-810 nm is obtained.
